Europäisches Patentamt

European Patent Office

Office européen des brevets

(11) Veröffentlichungsnummer: **0 365 817**
**A1**

## (12) EUROPÄISCHE PATENTANMELDUNG

(21) Anmeldenummer: 89117086.2

(22) Anmeldetag: **15.09.89**

(51) Int. Cl.5: **A61B 17/32 , A61B 17/39 , F16L 27/12**

(30) Priorität: **22.10.88 DE 3836120**

(43) Veröffentlichungstag der Anmeldung:
**02.05.90 Patentblatt 90/18**

(84) Benannte Vertragsstaaten:
**BE CH DE FR GB IT LI NL**

(71) Anmelder: **Richard Wolf GmbH**
**Pforzheimer Strasse 32**
**D-7134 Knittlingen(DE)**

(72) Erfinder: **Bauer, Siegfried**
**Vogesenstrasse 1**
**D-7521 Heidelsheim(DE)**
Erfinder: **Falk, Ernst**
**Meisenstrasse 13**
**D-7137 Sternenfels-Diefenbach(DE)**

(74) Vertreter: **Wilcken, Thomas, Dipl.-Ing. et al**
**Musterbahn 1**
**D-2400 Lübeck(DE)**

(54) **Resektoskop mit Schneidschlinge.**

(57) Das Resektoskop, bei dem durch den in den Schaft einsetzbaren Arbeitseinsatz parallel neben einer Optik ein Kanal (4) für eine axial verschiebbare Schneidschlinge verläuft, deren Schlingenschenkel (7) in einem Rohr (8) festgelegt sind und proximal in einen über eine Länge außerhalb des Schaftes frei bis zu einem Elektrodentransporteur (5) geführten Elektrodenstiel (9) übergeht, daß ein zylinderringförmiges Dichtungselement (10, 10a) aus Kunststoff auf dem Stiel (9) oder an der Innenseite des Kanals (4) befestigt ist, welches mit Dichtungsvorsprüngen (12,13,14, 15,16) gegen die Kanalinnenwand (4a) oder gegen den Außenumfang des Rohres (8) oder Stieles (9) der Schneidschlinge dichtend anliegen.

Fig. 8

## Resektoskop mit Schneidschlinge

Die Erfindung geht aus von einem Resektoskop , bei dem in einem Schaft ein eine Optik aufnehmender und einen Kanal für eine darin axial verschiebbare Schneidschlinge aufnehmender Arbeitseinsatz angeordnet ist.Die Schneidschlinge weist zwei sich proximalwärts erstreckende Schlingenschenkel auf, die in einem dünnwandigen Rohr festgelegt sind und proximal in einem Elektrodentransporteur lösbar festlegbaren Elektrodenstiel übergehen.

Dadurch, daß die an HF-STrom zu legende Schneidschlinge den Arbeitseinsatz proximalseits auf einer Teillänge bis zu dem Elektrodentransporteur freiliegt, kann am Ende des den Stiel der Schneidschlinge aufnehmenden im Arbeitseinsatz angeordneten Kanal ständig eine geringe Menge der in der Blase befindlichen Spülflüssigkeit unkontrolliert austreten. Dies führt dazu, daß der Operateur, der sich bei der Beobachtung der Resektion mit dem Gesicht in unmittelbarer Nähe der Flüssigkeitsaustrittsstelle befindet, einer Infektionsgefahr ausgesetzt ist.

Die Aufgabe der Erfindung besteht darin, das Austreten von Spülflüssigkeit am proximalen Ende des den Stiel der Schneidschlinge aufnehmenden Kanales bei Resektoskopen der vorerwähnten Art zu verhindern.

Diese Aufgabe wird durch den Anspruch 1 gelöst.Vorteilhaft geht man dabei so vor, daß das Dichtungselement mit dem distalen Ende in das die Schlingenschenkel festlegende Rohr eingreift und eine zylindrische Einschnürung besitzt, die mit den Dichtungsvorsprüngen,wie Lippen oder Noppen versehen ist, welche gegen die Innenwandung des den Stiel der Schneidschlinge aufnehmenden Kanals dichtend anliegen, ohne jedoch das Reibmoment bei der axialen Verschiebung zu erhöhen.

Man kann aber auch so vorgehen, daß auf der Innenseite des Kanals ein zylindrischer Dichtungsring aus Kunststoff befestigt ist, der mindestens eine nach innen gerichtete Lippe oder radial nach innen gerichtete Noppen zur dichtenden Anlage an das Rohr oder den Stiel der Schneidelektrode aufweist.

Das Dichtungselement für Resektoskope wird nachstehend anhand der Zeichnung erläutert, in der Ausführungsbeispiele dargestellt sind. Es zeigen:

Figur 1 die schematische Ansicht eines Arbeitseinsatzes mit Andeutung der Lage des Dichtungselementes,

Figur 2 bis 6 die Ansicht des Arbeitseinsatzes mit Ausführungsbeispielen des Dichtungselementes,

Figur 7 den Kupplungskegel des Arbeitseinsatzes mit einer ersten Ausführungsform des Dichtungselementes,

Figur 8 eine Schneidschlinge mit Anordnung eines Dichtungselementes

Figur 9 bis 13 Teilansichten der Schneidschlinge nach Fig. 8 mit Darstellung verschiedener Dichtungselemente.

Das Resektoskop besteht aus einem nicht dargestellten Schaft, einem Arbeitseinsatz 1,in den eine Optik 2 lösbar einsetzbar ist, und einen Kanal 4 für die Durchführung einer Schneidschlinge bzw. deren proximalseitiges Stielende.Der Arbeitseinsatz weist proximal einen axial verschiebbaren Elektroden-Transporteur 5 auf. Die Schneidschlinge 6 bis 10 besteht aus dem Schneidenteil 6, dessen beide Schenkel 7 durch ein Rohr 8 verlaufen und in dem Rohr 8 festgelegt sind. Diese Schenkel 7 gehen am proximalen Ende des Rohres 8 in einen Stiel 9 über, der mit dem Transporteur 5 verbunden und über ein Verbindungskabel an einen HF-Generator anschließbar ist.

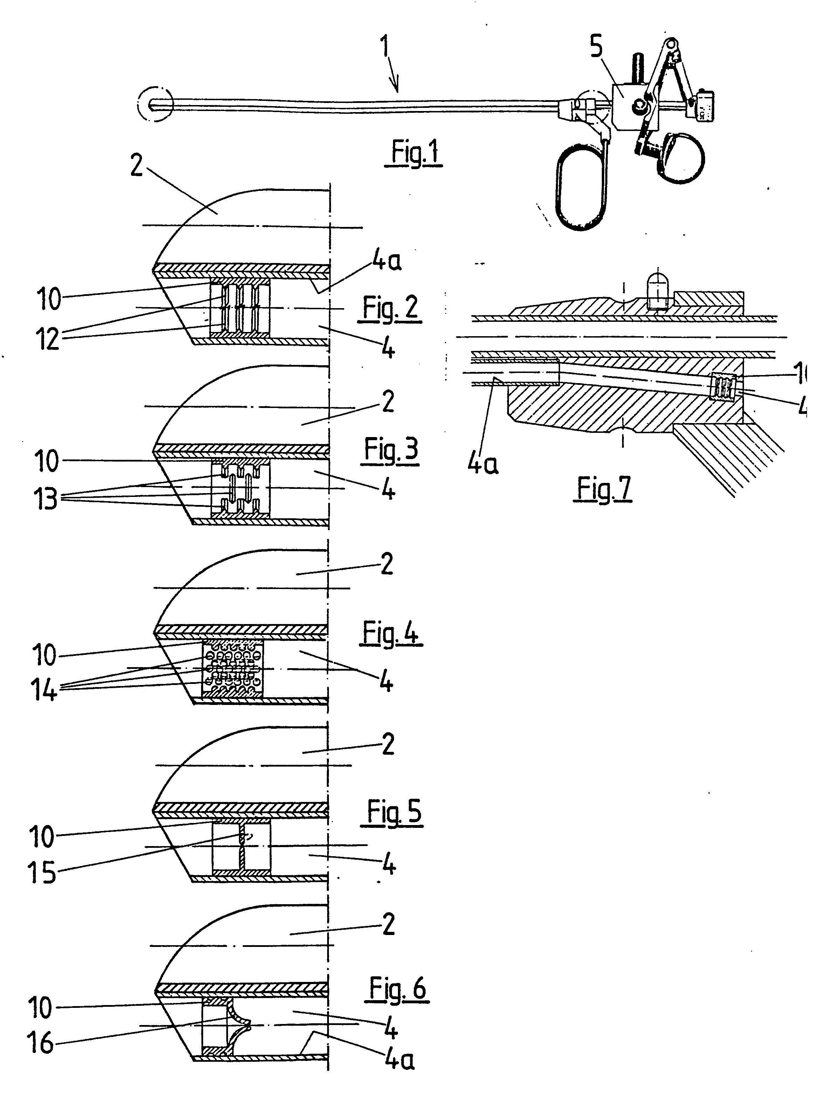

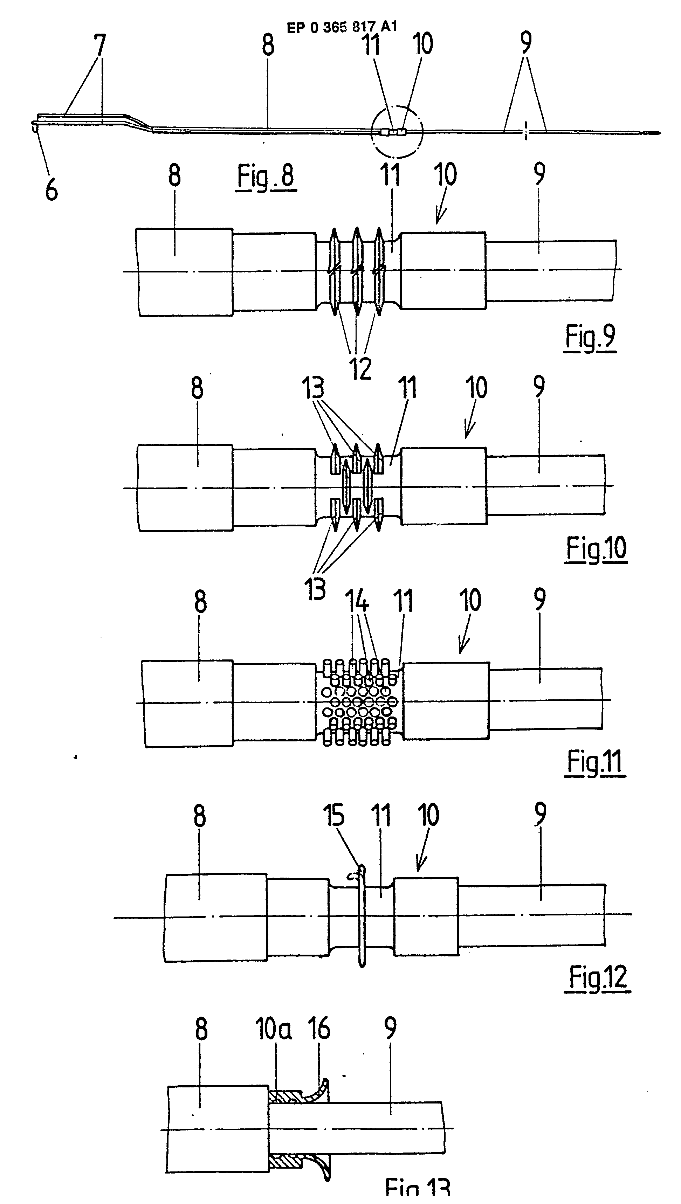

Nach den Figuren 2 bis 6 ist ein aus einem geeigneten Kunststoff bestehendes Dichtungselement 10 vorgesehen, welches zylinderringförmig ausgebildet ist und mit dem distalen Ende in das Rohr 8 sich festlegend eingreift. Dieses Dichtungselement 10 ist nach den Fig. 9 bis 12 mit einer zylindrischen Einschnürung 11 versehen.Nach Fig. 9 ist die Einschnürung 11 mit umlaufenden quer geschlitzten Dichtungslippen 12 versehen, deren Umfang den Umfang des Rohres 8 überragt und dichtend gegen die Innenwand des Kanals 4 anliegt.

Nach Fig.10 sind die Lippen 13 unterteilt und die Enden ihrer Längen liegen nahe beieinander und überlappen sich.

Nach Fig. 11 ist die Einschnürung 11 mit über den Umfang des Rohres 8 radial vorspringenden Noppen 14 versehen, die nahe beieinander liegen und sich durch Deformation berühren, sobald die Schneidschlinge den Kanal 4 eingeschoben wird.

Nach Fig. 12 ist die Einschnürung 11 mit nur einer Ringlippe 15 versehen, die die Abdichtung im Kanal 4 des Arbeitseinsatze bildet.

Nach Fig. 13 ist das Dichtungselement 10a mit dem distalen Ende wieder im Rohr 8 festgelegt und das proximale Ende als dichtende Ringlippe 16 ausgebildet.

Im Gegensatz zu den Figuren 9 bis 13 kann auch so vorgegangen werden, daß das zylinderringförmige Dichtungselement 10 an der Innenseite der Wandung 4a des Schaftkanals 4 befestigt ist, so daß dann die Lippen 12,13, die Noppen 14 und die Lippen 15 und 16 radial nach innen gerichtet sind und am distalen Ende des Kanals 4 gegen den Außenumfang des die Schenkel 7 der Schneidschlinge festlegenden Rohres 8 dichtend zur Anlage kommen.

Nach Fig. 7 kann das Dichtungselement 10 auch im proximalen Ende des Kanals 4 angeordnet werden.

Die Ausführungen nach Fig. 2 bis 6 haben den Vorteil, daß die bisher produzierten und im Handel befindlichen Schneidschlingen ohne Änderung weiter verwendbar sind.

Da Schneidschlingen als Verbrauchsartikel anzusehen sind, dürfte die Ausführung nach den Fig. 9 bis 13 günstiger sein, da dann die Abdichtung während der gesamten Gebrauchszeit des Einsatzes gewährleistet ist.

## Ansprüche

1. Resektoskop, bei dem durch einen Schaft parallel neben einer Optik ein Kanal für einen axial verschiebbaren Arbeitseinsatz mit Schneidschlinge verläuft, deren Schlingenschenkel in einem Rohr des Arbeitseinsatzes festgelegt sind und proximal in einen über eine Länge außerhalb des Schaftes frei bis zu einem Elektrodentransporteur geführten Elektrodenstiel übergeht, dadurch gekennzeichnet, daß ein zylinderringförmiges Dichtungselement (10,10a) aus Kunststoff auf dem Stiel (9) oder an der Innenseite des Kanals (4) befestigt ist, welches mit Dichtungsvorsprüngen (12,13,14,15,16) gegen die Kanalinnenwand (4a) oder gegen den Außenumfang des Rohres(8) oder Stieles (9) des Arbeitseinsatzes dichtend anliegen.

2. Resektoskop nach Anspruch 1, dadurch gekennzeichnet, daß das Dichtungselement (10) mit dem distalen Ende in das die Schlingenschenkel (7) festlegende Rohr (8) eingreift und eine zylindrische Einschnürung (11) aufweist,die mit den Dichtungsvorsprüngen , wie Lippen (12) oder Noppen (14) versehen ist, welche gegen die Innenwand des Kanals (4)dichtend anliegen.

3. Resektoskop nach Anspruch 2,dadurch gekennzeichnet, daß die umlaufenden Dichtungslippen (12) quer geschlitzt sind.

4. Resektoskop nach Anspruch 2, dadurch gekennzeichnet, daß die Dichtungslippen (13) unterteilt sind und Teilringe darstellen, die sich mit den Enden überlappen.

5. Resektoskop nach Anspruch 1 und 2, dadurch gekennzeichnet, daß die Einschnürung (11) des Dichtungselementes (10) mit nahe aneinander liegenden radial abstehenden Dichtungsnoppen (14) versehen ist.

6. Resektoskop nach Anspruch 1, dadurch gekennzeichnet, daß das Dichtungselement (10a) aus einem Kunststoffring besteht, der mit einem distalen Längenteil in das proximale Ende des die Schlingenschenkel festlegenden Rohres (8) eingreifend festgelegt ist und der eine ringförmige Dichtungslippe (16)aufweist.

7. Resektoskop nach Anspruch 1, dadurch gekennzeichnet, daß auf der Innenseite des Kanals (4) ein zylindrischer Dichtungsring (10) aus Kunststoff befestigt ist, der mindestens eine nach innen gerichtete Lippe (12, 13,15,16) oder nach innen gerichtete radiale Noppen (14) zur dichtenden Anlage an das Rohr (8) oder an den Stiel (9) der Schneidelektrode aufweist.

**Fig.1**

**Fig.2**

**Fig.3**

**Fig.4**

**Fig.5**

**Fig.6**

**Fig.7**

EP 0 365 817 A1

Fig. 8

Fig. 9

Fig. 10

Fig. 11

Fig. 12

Fig. 13

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 185 810  (HAKKY)<br>* Seite 10, Zeile 1 - Seite 11, Zeile 15; Zusammenfassung; Figur 1 *<br>--- | 1,7 | A 61 B  17/32<br>A 61 B  17/39<br>F 16 L  27/12 |
| Y | DE-A-2 817 922  (OLYMPUS)<br>* Seite 11, Zeile 11 - Seite 12, Zeile 25; Figur 3 * | 1,7 | |
| A | | 2,6 | |
| A | DE-A-3 735 945  (OLYMPUS)<br>* Spalte 3, Zeilen 2-15; Figuren 2,9(b) *<br>--- | 1 | |
| A | DE-A-3 601 453  (SACHSE)<br>* Spalte 5, Zeilen 26,27; Figur 1 *<br>--- | 1 | |
| A | FR-A-1 390 825  (U.S.A.E.C.)<br>* Seite 1, Spalte 2, Zeilen 4-8,18-20; Figur *<br>--- | 2,7 | |
| A | DE-A-1 922 819  (WUNDERLICH)<br>* Seite 6, Zeilen 14-19; Figuren 1-5,7,9 *<br>--- | 7 | **RECHERCHIERTE SACHGEBIETE (Int. Cl.5)**<br><br>A 61 B<br>A 61 F<br>F 16 L |
| A | DE-A-3 707 403  (OLYMPUS)<br>--- | | |
| A | DE-B-1 029 632  (HAMACHER)<br>--- | | |
| A | US-A-4 702 671  (BRINKMAN)<br>----- | | |

Der vorliegende Recherchenbericht wurde für alle Patentansprüche erstellt

| Recherchenort | Abschlußdatum der Recherche | Prüfer |
|---|---|---|
| DEN HAAG | 13-12-1989 | KLEIN C. |

EPO FORM 1503 03.82 (P0403)